# EUROPEAN PATENT APPLICATION

(11) **EP 4 174 181 A1**
(43) Date of publication of application: **03.05.2023**
(21) Application number: 21829053.4
(22) Date of filing: 24.06.2021
(51) Int. Cl.: C12N 15/52, C12N 15/36, C12N 15/867, C12N 15/65, C12N 5/10, C12Q 1/02, C12Q 1/66

(54) **DRUG SCREENING MODEL AND METHOD FOR TARGETING HBV CCCDNA**

(30) Priority: 24.06.2020 CN 202010588643
(71) Applicant: Xiamen University, Xiamen, Fujian 361005 (CN); Yang Sheng Tang Company, Ltd., Hangzhou, Zhejiang 310000 (CN)
(72) Inventor: YUAN, Quan, Xiamen, Fujian 361005 (CN); CAO, Jiali, Xiamen, Fujian 361005 (CN); ZHANG, Yali, Xiamen, Fujian 361005 (CN); WANG, Mingfeng, Xiamen, Fujian 361005 (CN); MA, Jian, Xiamen, Fujian 361005 (CN); ZHANG, Tianying, Xiamen, Fujian 361005 (CN); ZHANG, Jun, Xiamen, Fujian 361005 (CN); XIA, Ningshao, Xiamen, Fujian 361005 (CN)
(74) Representative: Meissner Bolte Partnerschaft mbB
(86) International application number: PCT/CN2021/101986
(87) International publication number: WO 2021/259345

(57) **Abstract**

The present invention belongs to the field of virology, in particular to the field of hepatitis B virus treatment. Provided are a model and a method for screening HBV cccDNA inhibitors. According to the screening model and the method, the detection of a split luciferase is used as an alternative index of HBV cccDNA detection, and a cccDNA-targeted drug can be screened in high throughput.

## Description

### Technical Field

The present invention relates to the field of virology, in particular to the field of hepatitis B virus treatment. In particular, the present invention relates to a model and method for screening HBV cccDNA inhibitors.

### Background Art

Chronic hepatitis B (CHB) caused by hepatitis B virus (HBV) is one of the most serious public health problems in the world. More than 800,000 people die each year from various liver diseases caused by hepatitis B virus infection, including chronic active hepatitis, liver cirrhosis and hepatocellular carcinoma. At present, the two main types of therapeutic drugs (nucleoside analogs and interferons) in clinical practice are difficult to achieve clinical cure. The stable existence of HBV cccDNA is one of the key reasons why chronic hepatitis B is difficult to cure. At present, no clinical drugs can effectively eliminate cccDNA, and the cccDNA existing in the cell can continue to serve as a template for virus replication and transcription.

Due to the complex mechanism of HBV cccDNA formation and maintenance, it is highly difficult to directly design a drug against cccDNA. A screening model that can be used for high-throughput screening of cccDNA inhibitors provides a new method for developing drugs that can eliminate cccDNA. The detection method of cccDNA is complex. The Southern blot is the gold standard for cccDNA detection, but it requires a large amount of cells, with complicated operation, and is time-consuming, so it cannot be used for high-throughput drug screening. Compared with the Southern blot, fluorescence quantitative PCR detection is simpler, faster, and has higher throughput, but it is also difficult to apply to large-scale drug screening, and the detection may be interfered by rcDNA. Using markers that are easier to detect as surrogate markers for cccDNA detection can reduce detection costs, improve detection efficiency, and improve detection throughput.

The ideal cccDNA reporter model should not only satisfy the requirement of stable source of cccDNA, but also satisfy the requirements of easy detection of the surrogate detection marker with high signal-to-noise ratio. Therefore, it is necessary to develop an HBV cccDNA reporter model suitable for high-throughput screening.

### Contents of the Invention

After a lot of experiments and repeated explorations, the inventors of the present application have constructed an HBV cccDNA reporter model using split luciferase as a surrogate indicator for HBV cccDNA detection, which is simple in operation, short time-consuming, and can achieve high-throughput drug screening. Therefore, this model can be used for preliminary screening for candidate drugs with inhibitory potential to HBV cccDNA which can be further verified in *in vitro* and *in vivo* research models of HBV.

### Reporter Model I

In some cases, a first fragment sequence (e.g., HiBiT) in luciferase fragment complementation assay (LFCA) can be integrated into the HBV genome to form an HBV variant, an mRNA transcribed from the HBV variant as template lacks the initiation codon for the expression of the first fragment (e.g., HiBiT) and thus cannot translate a protein attached to the first fragment (e.g., HiBiT) tag. Only after cccDNA is formed by reverse transcription of the pgRNA transcribed from the HBV variant, the mRNA transcribed from the cccDNA as template can translate the protein attached to the first fragment (e.g., HiBiT) tag. Thus, the expression level of the first fragment (e.g., HiBiT) can be measured by the luciferase fragment complementation assay (LFCA), thereby indicating the formation of HBV cccDNA.

### 1. Isolated nucleic acid molecules

Accordingly, in a first aspect, the present invention provides an isolated nucleic acid molecule, which comprises a variant of HBV genome sequence (e.g., wild-type HBV genome), the variant comprises: an HBV genome fragment comprising C-ORF, S-ORF and P-ORF, the C-ORF comprises an exogenous insertion sequence between the precore and core genes, and the exogenous insertion sequence comprises a nucleotide sequence encoding a first fragment of luciferase. The first fragment of luciferase is capable of binding to a corresponding second fragment of luciferase in the luciferase fragment complementation assay (LFCA) to generate luciferase activity.

As used herein, the term "luciferase fragment complementation assay (LFCA)" has the meaning commonly understood by those skilled in the art, which divides luciferase into a first fragment and a second fragment that are each enzymatically inactive, when the two fragments are interacted between each other, they can complement each other and generate luciferase activity, thereby releasing a luminescent signal in the presence of a luciferase substrate. In certain exemplary embodiments, the luciferase fragment complementation assay is based on LgBiT and a complementary small fragment (e.g., HiBiT or SmBiT) capable of binding thereto from Promega Corporation, in which a functional enzyme will be generated through the structural complementation of LgBiT with HiBiT or SmBiT.

In certain embodiments, the first fragment of luciferase is LgBiT and the second fragment of luciferase is a complementary small fragment (e.g., HiBiT or SmBiT) capable of binding to LgBiT.

In certain embodiments, the first fragment of luciferase is a complementary small fragment (e.g., HiBiT or SmBiT) capable of binding to LgBiT, and the second fragment of luciferase is LgBiT. In certain embodiments, the first fragment of luciferase is HiBiT and the second fragment of luciferase is LgBiT. In certain embodiments, HiBiT has the sequence set forth in SEQ ID NO:2. In certain embodiments, the nucleotide sequence encoding HiBiT is set forth in SEQ ID NO:3.

In certain embodiments, the HBV genome fragment further comprises an X-ORF.

In certain embodiments, the variant comprises the exogenous insertion sequence between the precore and core genes of an HBV genome sequence (e.g., a wild-type HBV genome).

In certain embodiments, the exogenous insertion sequence comprises multiple copies of the nucleotide sequence encoding the first fragment of luciferase (e.g., HiBiT) in tandem repeats. In certain embodiments, the exogenous insertion sequence comprises three copies of the nucleotide sequence encoding the first fragment of luciferase (e.g., HiBiT) in tandem repeats.

In certain embodiments, each copy of the multiple copies of the nucleotide sequence encoding the first fragment of luciferase (e.g., HiBiT) in tandem repeats comprises a sequence encoding a linker peptide at its 5' end. In certain embodiments, the linker peptide is a flexible peptide linker. In certain embodiments, the linker peptide consists of G (glycine) and/or S (serine). In certain embodiments, the linker peptide is GSG.

In certain embodiments, the exogenous insertion sequence comprises the sequence set forth in SEQ ID NO:4.

In certain embodiments, the HBV genome is a full-length genome, for example, a genome of HBV genotype A, B, C, D, E, F, G or H. In certain embodiments, the HBV genome is an overlength genome, for example, a 1.1-fold genome or a 1.3-fold genome. In certain embodiments, the HBV genome is a 1.1-fold genome, for example, as set forth in SEQ ID NO: 1.

In certain embodiments, the exogenous insertion sequence is operably linked to an inducible promoter.

In certain embodiments, the exogenous insertion sequence is regulated for expression by the Tet-On gene expression system. Therefore, in certain embodiments, the inducible promoter is Tet operator (TetO) or promoter in the Tet-On gene expression system, which requires Doxycycline to bind to its corresponding transactivator to initiate transcription.

In certain embodiments, the inducible promoter is a TRE3G promoter (e.g., as set forth in SEQ ID NO: 5) and the corresponding transactivator is a Tet-On 3G transactivator (e.g., as set forth in SEQ ID NO: 9).

In certain embodiments, the inducible promoter is one or more repeats of a Tet operator (TetO) sequence, and the corresponding transactivator may be a reverse Tet repressor (rTetR) or reverse Tet transcription activator (rtTA).

In certain embodiments, the inducible promoter has bidirectional promoter activity.

In certain embodiments, the inducible promoter is a TRE3G promoter with bidirectional promoter activity.

In certain embodiments, the inducible promoter is operably linked to a reporter gene. In certain embodiments, the reporter gene is oriented opposite to the exogenous insertion sequence. In certain embodiments, the reporter gene is selected from fluorescent protein genes and/or antibiotic resistance genes.

In certain embodiments, the fluorescent protein is selected from the group consisting of green fluorescent protein, blue fluorescent protein, cyan fluorescent protein, yellow fluorescent protein, orange or red fluorescent protein, near-infrared fluorescent protein, or long Stokes shift fluorescent protein. In certain embodiments, the fluorescent protein is selected from the group consisting of red fluorescent protein, near-infrared fluorescent protein, or long Stokes shift fluorescent protein, such as mRuby3, mApple, FusionRed, mCherry, mScarlet, RFP, iRFP670, mBeRFP, or CyOFP1. In certain embodiments, the fluorescent protein is selected from the group consisting of green fluorescent proteins, for example, mGamillus, mNeonGreen, EGFP, mClover, UnaG, TurboGFP, TagGFP, Venus, EYFP, RFP, iRFP670, mBeRFP, CyOFP1.

In certain embodiments, the antibiotic resistance gene is selected from the group consisting of genes capable of conferring resistance to hygromycin, neomycin, G418, blasticidin, puromycin or ouabain.

In certain embodiments, the reporter gene comprises a fluorescent protein gene and an antibiotic resistance gene. In certain embodiments, the reporter gene comprises a gene encoding an iRFP (e.g., as set forth in SEQ ID NO: 11) and a Blasticidin resistance gene (e.g., as set forth in SEQ ID NO: 13).

In certain embodiments, the fluorescent protein gene and the antibiotic resistance gene are optionally linked by a nucleotide sequence encoding a self-cleaving peptide (e.g., P2A, E2A, F2A or T2A). In certain embodiments, the cleavage peptide is P2A, for example, as set forth in SEQ ID NO:6.

In certain embodiments, the isolated nucleic acid molecule comprises the sequence set forth in SEQ ID NO:8.

### 2. Recombinant HBV cccDNA

The HBV variant contained in the isolated nucleic acid molecule described in the first aspect of the present invention can be transcribed as a template to form pgRNA, and reversely transcribed to form cccDNA.

Therefore, in a second aspect, the present invention also provides a recombinant HBV cccDNA, which comprises the isolated nucleic acid molecule of the first aspect.

In certain embodiments, the recombinant HBV cccDNA comprises a variant of the HBV genome sequence described in the first aspect.

In certain embodiments, the recombinant HBV cccDNA is formed by circularization of the isolated nucleic acid molecule of the first aspect.

### 3. Expression system

In a third aspect, the present invention provides an expression system, which comprises the isolated nucleic acid molecule of the first aspect.

In certain embodiments, the isolated nucleic acid molecule comprises an inducible promoter operably linked to an exogenous insertion sequence, and the expression system comprises the isolated nucleic acid molecule as a first nucleic acid sequence and comprises a second nucleic acid sequence, the second nucleic acid sequence comprises a nucleotide sequence encoding a transactivator corresponding to the inducible promoter.

In certain embodiments, the transactivator is selected from the group consisting of Tet-On 3G transactivator, rTetR, rtTA.

In certain embodiments, the second nucleic acid sequence further comprises an expression control element, such as a promoter (e.g., a constitutive promoter) and/or enhancer, operably linked to the nucleotide sequence encoding the transactivator.

In certain embodiments, the first nucleic acid sequence comprises a TRE3G promoter as the inducible promoter, and the second nucleic acid sequence comprises a nucleotide sequence encoding Tet-On 3G transactivator. In certain embodiments, the TRE3G promoter comprises the sequence set forth in SEQ ID NO:5. In certain embodiments, the nucleotide sequence encoding Tet-On 3G transactivator comprises the sequence set forth in SEQ ID NO: 10.

### 4. Vector

In a fourth aspect, the present invention also provides a vector, which comprises the isolated nucleic acid molecule of the first aspect, or the expression system of the third aspect.

In certain embodiments, the vector comprises the expression system of the third aspect, wherein the first nucleic acid sequence and the second nucleic acid sequence are provided on the same or different vectors. In certain embodiments, the first nucleic acid sequence and the second nucleic acid sequence are provided on the same vector.

In certain embodiments, the vector is a transposon vector, such as a PiggyBac transposon vector. In certain embodiments, the first nucleic acid sequence and/or the second nucleic acid sequence can be inserted into any commercially available PiggyBac transposon vector, such as PB-CMV-MCS-EF1α-RedPuro (Cat.# PB514B-1). In certain embodiments, the first nucleic acid sequence and the second nucleic acid sequence are located between two ITR sequences of the transposon vector.

### 5. Co-transfection system

In a fifth aspect, the present invention provides a co-transfection system, which comprises the vector of the fourth aspect, wherein the vector is a transposon vector, and a transposase expression vector.

In certain embodiments, the transposase expression vector is a PiggyBac transposase expression vector. Herein, the PiggyBac transposase expression vector is well known in the art and is widely commercially available. In certain embodiments, the PiggyBac transposase expression vector is PB210PA-1 (System Biosciences).

### 6. Host cell

In a sixth aspect, the present invention provides a host cell, which comprises the isolated nucleic acid molecule of the first aspect, or the recombinant HBV cccDNA of the second aspect, or the expression system of the third aspect, or the vector of the fourth aspect, or the co-transfection system of the fifth aspect.

In certain embodiments, the host cell is an eukaryotic cell. In certain embodiments, the host cell supports formation and transcription of functional HBV cccDNA.

In certain embodiments, the host cell is an eukaryotic cell of hepatocyte origin, such as hepatoma cell or hepatocyte. In certain embodiments, the host cell is selected from HepaRG, HepG2 or Huh7.

In certain embodiments, the host cell may also be non-hepatocyte, provided that it supports cccDNA formation of hepadnavirus (or in a broader sense, DNA replication of hepadnavirus). For example, if viral pregenomic RNA is introduced into a cell or transcribed from a DNA template via an exogenous promoter, such non-hepatocyte/host can be modified to support cccDNA formation of hepadnavirus (or DNA replication of hepadnavirus).

In certain embodiments, the host cell comprises the expression system of the third aspect in its genome.

In certain embodiments, the host cell is capable of stably expressing the HBV cccDNA formed by the variant of HBV genome sequence in the presence of an inducer (e.g., Doxycycline) corresponding to the inducible promoter and transactivator.

### 7. Kit

In a seventh aspect, the present invention provides a kit, which comprises the isolated nucleic acid molecule of the first aspect, or the expression system of the third aspect, or the vector of the fourth aspect, or the co-transfection system of the fifth aspect, or the host cell of the sixth aspect.

In certain embodiments, the kit comprises: the vector of the fourth aspect, or the co-transfection system of the fifth aspect.

In certain embodiments, the kit comprises: the host cell of the sixth aspect.

In certain embodiments, the kit further comprises LgBiT protein. Optionally, the kit may also comprise a luciferase substrate.

In certain embodiments, the kit further comprises an inducer (e.g., Doxycycline) corresponding to the inducible promoter and transactivator.

### 8. Screening method

In an eighth aspect, provided is a method for screening HBV cccDNA inhibitor, comprising:
(1) providing the host cell of the sixth aspect; the host cell comprises the expression system described in the third aspect in its genome;
(2) contacting an inducing agent with the host cell, the inducing agent is an inducer (e.g., Doxycycline) corresponding to the inducible promoter and transactivator contained in the host cell;
(3) contacting a test agent with the host cell; wherein, steps (2) and (3) can be performed simultaneously or in any order;
(4) detecting a level of the first fragment of luciferase (e.g., HiBiT) in a cell supernatant of the host cell.

In certain embodiments, step (1) comprises the steps of:
(1a) introducing the first nucleotide sequence and the second nucleotide sequence in the expression system of the third aspect into the host cell, wherein the first nucleotide sequence and the second nucleotide sequence are provided on the same or different expression vectors, and the first nucleic acid sequence is an isolated nucleic acid molecule as described in the first aspect comprising an inducible promoter operably linked to the exogenous insertion sequence;
(1b) culturing the host cell.

In certain embodiments, the host cell is selected from hepatocyte-derived eukaryotic cells, such as hepatoma cells or hepatocytes; preferably, the host cell is selected from HepaRG, HepG2 or Huh7.

In certain embodiments, in step (1a), the expression vector is a transposon vector (e.g., a PiggyBac transposon vector), and the step further comprises: introducing a transposase expression vector (e.g., a PiggyBac transposase expression vector) into the host cell.

In certain embodiments, the step (1) further comprises: (1c) identifying and selecting a host cell that has integrated the expression system of the third aspect into its genome. In certain embodiments, whether the expression system has been integrated into the genome of the host cell is identified by detecting a reporter gene contained in the first nucleic acid sequence.

In certain embodiments, in step (2), the inducing agent activates the inducible promoter, thereby initiating transcription and replication of the HBV genome variant downstream thereof, resulting in a recombinant HBV cccDNA, the recombinant HBV cccDNA comprising the first fragment of luciferase as a label.

In certain embodiments, the step (2) comprises culturing the host cell under conditions that permit: (i) synthesis of HBV pregenomic (pg) RNA; (ii) reverse transcription of the synthesized pgRNA into a negative-strand DNA; (iii) synthesis of a second positive-strand DNA so that the negative-strand DNA and the positive-strand DNA form double-stranded relaxed circular DNA; (iv) formation of cccDNA from the double-stranded relaxed circular DNA.

In certain embodiments, in step (4), the level of the first fragment of luciferase is detected by luciferase fragment complementation assay (i.e., by providing a second fragment of luciferase that is structurally complementary to the first fragment, and a luciferase substrate).

In certain embodiments, a second fragment of luciferase that is complementary to the first fragment of luciferase is used for detection.

In certain embodiments, the first fragment of luciferase is a complementary small fragment capable of binding to LgBiT, such as HiBiT or SmBiT, and the second fragment of luciferase is an LgBiT protein. In certain embodiments, the first fragment of luciferase is HiBiT or SmBiT, and the second fragment of luciferase is an LgBiT protein.

In certain embodiments, the method further comprises the steps of:
(5) comparing the detection result of step (4) with the level of first fragment of luciferase detected in the absence of the test agent; wherein, if the detection result of step (4) is lower than the detection result obtained in the absence of the test agent, it indicates that the test agent is an HBV cccDNA inhibitor.

The present invention also relates to use of the isolated nucleic acid molecule, expression system, vector, co-transfection system, host cell, and kit described above for screening an HBV cccDNA inhibitor.

### Reporter Model II

In other cases, a linear HBV replicon can be circularized to form cccDNA using recombinase technology. The linear HBV replicon comprises a first fragment sequence (e.g., HiBiT sequence) of luciferase fragment complementation assay (LFCA) integrated therein. Without expression of recombinase, the first fragment (e.g., HiBiT) tag lacks a promoter and thus cannot be expressed; while with expression of recombinase, it can mediate the recombination of double-stranded DNA, so that the linear HBV genome DNA forms a closed circular DNA. After the circular DNA is formed, the first fragment (e.g., HiBiT) tag can utilize an HBV endogenous promoter to initiate the expression of protein attached to the first fragment (e.g., HiBiT) tag. Therefore, the signal of the first fragment (e.g., HiBiT) tag can be determined by luciferase fragment complementation assay (LFCA) to indicate the formation of recombinant HBV cccDNA, i.e., HBV rcccDNA.

### 9. Isolated nucleic acid molecule

Accordingly, in a ninth aspect, the present invention provides an isolated nucleic acid molecule, which comprises a variant of HBV genome sequence (e.g., a wild-type HBV genome), and the variant comprises, from 5' to 3':
(i) a nucleotide sequence encoding a first fragment of luciferase; in which the first fragment of luciferase is capable of binding to a corresponding second fragment of luciferase in the luciferase fragment complementation assay (LFCA) to generate luciferase activity;
(ii) a sequence of the 3' end region of C-ORF of HBV genome;
(iii) an HBV genome fragment containing S-ORF and P-ORF;
(iv) a sequence of the 5' end region of C-ORF of HBV genome, which can form a complete C-ORF sequence with the sequence described in (ii);
and the variant is located between two site-specific recombinase recognition sequences arranged in the same orientation.

In certain embodiments, the first fragment of luciferase is a complementary small fragment capable of binding to LgBiT, such as HiBiT or SmBiT, and the second fragment of luciferase is LgBiT. In certain embodiments, the first fragment of luciferase is HiBiT and the second fragment of luciferase is LgBiT. In certain embodiments, HiBiT has the sequence set forth in SEQ ID NO:2. In certain embodiments, the nucleotide sequence encoding HiBiT is set forth in SEQ ID NO:3.

In certain embodiments, the HBV genome is a full-length genome, for example, a genome of HBV genotype A, B, C, D, E, F, G or H. In certain embodiments, the HBV genome is an overlength genome, for example, a 1.1-fold genome or a 1.3-fold genome. In certain embodiments, the HBV genome is a 1.1-fold genome, for example, as set forth in SEQ ID NO: 1.

In certain embodiments, the sequence of (iii) further comprises an X-ORF.

In certain embodiments, the sequence of (iii) comprises a HBV genome fragment with C-ORF removed.

In certain embodiments, the sequence of (iii) comprises the sequence set forth in SEQ ID NO: 16.

In certain embodiments, the sequence of (ii) comprises a core gene and the sequence of (iv) comprises a pre-core gene. In certain embodiments, the sequence of (ii) comprises the sequence set forth in SEQ ID NO: 14. In certain embodiments, the sequence of (iv) comprises the sequence set forth in SEQ ID NO: 15.

In certain embodiments, the site-specific recombinase recognition sequence is selected from a loxP sequence or a FRT sequence.

In certain embodiments, the isolated nucleic acid molecule comprises the sequence set forth in SEQ ID NO: 17.

### 10. Recombinant HBV cccDNA

The isolated nucleic acid molecule according to the ninth aspect of the present invention can be circularized under the action of recombinase to form cccDNA.

Accordingly, in a tenth aspect, the present invention also provides a recombinant HBV cccDNA, which is formed by circularization of the variant of HBV genome sequence contained in the isolated nucleic acid molecule of the ninth aspect.

In certain embodiments, the recombinant HBV cccDNA is formed by circularization of the isolated nucleic acid molecule of the ninth aspect in the presence of a site-specific recombinase (e.g., Cre recombinase or FLP recombinase) corresponding to the site-specific recombinase recognition sequence.

In certain embodiments, the recombinant HBV cccDNA comprises C-ORF, S-ORF, P-ORF, and the C-ORF comprises a nucleotide sequence encoding the first fragment of luciferase (e.g., HiBiT).

In certain embodiments, the recombinant HBV cccDNA further comprises an X-ORF.

In certain embodiments, the recombinant HBV cccDNA comprises: a C-ORF comprising a nucleotide sequence encoding the first fragment of luciferase (e.g., HiBiT), and an HBV genome fragment from which the C-ORF has been removed (e.g., the sequence set forth in SEQ ID NO: 16).

In certain embodiments, the recombinant cccDNA comprises the sequence set forth in SEQ ID NO:18.

### 11. Vector

In an eleventh aspect, the present invention also provides a vector comprising the isolated nucleic acid molecule of the ninth aspect.

In certain embodiments, the vector is a transposon vector, such as a PiggyBac transposon vector. In certain embodiments, the isolated nucleic acid molecule of the ninth aspect can be inserted into any commercially available PiggyBac transposon vector, such as PB-CMV-MCS-EF1α-RedPuro (Cat. #PB514B-1). In certain embodiments, the isolated nucleic acid molecule is located between two ITR sequences of the transposon vector.

### 12. Co-transfection system

In the twelfth aspect, the present invention also provides a co-transfection system, which comprises the vector described in the eleventh aspect, and a transposase expression vector.

In certain embodiments, the transposase expression vector is a PiggyBac transposase expression vector. Herein, the PiggyBac transposase expression vector is well known in the art and is widely commercially available. In certain embodiments, the PiggyBac transposase expression vector is PB210PA-1 (System Biosciences).

### 13. Host cell

In a thirteenth aspect, the present invention provides a host cell, which comprises the isolated nucleic acid molecule of the ninth aspect, or the recombinant cccDNA of the tenth aspect, or the vector of the eleventh aspect, or the co-transfection system of the twelve aspect.

In certain embodiments, the host cell is an eukaryotic cell. In certain embodiments, the host cell supports formation and transcription of functional HBV cccDNA.

In certain embodiments, the host cell is an eukaryotic cell derived from hepatocytes, such as hepatoma cells or hepatocytes. In certain embodiments, the host cell is selected from HepaRG, HepG2 or Huh7.

In certain embodiments, the host cell may also be a non-hepatocyte, provided that it supports cccDNA formation of hepadnavirus (or in a broader sense, DNA replication of hepadnavirus). For example, if viral pregenomic RNA is introduced into a cell or transcribed from a DNA template via an exogenous promoter, such non-hepatocyte/host can be modified to support cccDNA formation of hepadnavirus (or DNA replication of hepadnavirus).

In certain embodiments, the host cell comprises the isolated nucleic acid molecule of the ninth aspect in its genome.

In certain embodiments, when a site-specific recombinase (e.g., Cre recombinase or FLP recombinase) corresponding to the site-specific recombinase recognition sequence exists, the host cell is capable of stably expressing the recombinant HBV cccDNA formed by circularization of the variant of HBV genome sequence.

### 14. Kit

In a fourteenth aspect, the present invention provides a kit, which comprises the isolated nucleic acid molecule of the ninth aspect, or the recombinant cccDNA of the tenth aspect, or the vector of the eleventh aspect, or the co-transfection system of the twelfth aspect, or the host cell of the thirteenth aspect.

In certain embodiments, the kit comprises: the vector of the eleventh aspect, or the co-transfection system of the twelfth aspect.

In certain embodiments, the kit comprises: the host cell of the thirteenth aspect.

In certain embodiments, the kit further comprises LgBiT protein. Optionally, the kit further comprises a luciferase substrate.

In certain embodiments, the kit further comprises a recombinase (e.g., Cre recombinase or FLP recombinase) or recombinase (e.g., Cre recombinase or FLP recombinase) expression vector.

### 15. Screening method

In a fifteenth aspect, the present invention provides a method for screening an HBV cccDNA inhibitor, comprising:
(1) providing the host cell of the thirteenth aspect; the host cell comprises the isolated nucleic acid molecule of the ninth aspect in its genome;
(2) introducing a recombinase or a recombinase expression vector into the host cell, the recombinase corresponds to the site-specific recombinase recognition sequence contained in the host cell;
(3) contacting a test agent with the host cell;
(4) detecting a level of first fragment of luciferase (e.g., HiBiT) in a cell supernatant of the host cell.

In certain embodiments, step (1) comprises the steps of:
(1a) introducing the isolated nucleic acid molecule described in the ninth aspect or the vector described in the eleventh aspect into the host cell;
(1b) culturing the host cell.

In certain embodiments, the host cell is selected from hepatocyte-derived eukaryotic cells, such as hepatoma cells or hepatocytes; preferably, the host cell is selected from HepaRG, HepG2 or Huh7.

In certain embodiments, in step (1a), the expression vector is a transposon vector (e.g., a PiggyBac transposon vector), and the step further comprises: introducing a transposase expression vector (e.g., a PiggyBac transposase expression vector) into the host cell.

In certain embodiments, in step (2), the variant of HBV genome sequence contained in the host cell will be circularized to form cccDNA under the action of a recombinase.

In certain embodiments, in step (4), the level of first fragment of luciferase is detected by the luciferase fragment complementation assay (i.e., by providing a second fragment of luciferase that is structurally complementary to the first fragment, and a luciferase substrate).

In certain embodiments, a second fragment of luciferase that is complementary to the first fragment of luciferase is used for detection.

In certain embodiments, the first fragment of luciferase is a complementary small fragment capable of binding to LgBiT, such as HiBiT or SmBiT, and the second fragment of luciferase is an LgBiT protein. In certain embodiments, the first fragment of luciferase is HiBiT and the second fragment of luciferase is an LgBiT protein.

In certain embodiments, the method further comprises the steps of:
(5) comparing the detection result of step (4) with the level of first fragment of luciferase detected in the absence of the test agent; wherein, if the detection result of step (4) is lower than the detection result obtained in the absence of the test agent, it indicates that the test agent is an HBV cccDNA inhibitor.

The present invention also relates to use of the isolated nucleic acid molecule, vector, co-transfection system, host cell, and kit described above for screening an HBV cccDNA inhibitor.

### Definition of Term

In the present invention, unless otherwise specified, scientific and technical terms used herein have the meanings commonly understood by those skilled in the art. In addition, the laboratory procedures of virology, cell culture, biochemistry, nucleic acid chemistry, immunology, etc. used herein are all routine steps widely used in the corresponding fields. Meanwhile, for a better understanding of the present invention, definitions and explanations of related terms are provided below.

As used herein, the term "hepatitis B virus (HBV)" refers to a member of the *Hepadnaviridae* family with a small double-stranded DNA genome of approximately 3200 base pairs and hepatocyte tropism. "HBV" includes any hepatitis B virus that infects any of a variety of hosts of mammalian (e.g., human, non-human primate, etc.) and avian (duck, etc.). "HBV" includes any known HBV genotype, such as serotypes A, B, C, D, E, F and G; any HBV serotype or HBV subtype; any HBV isolate; HBV variant, such as HBeAg negative variant, drug-resistant HBV variant (e.g., lamivudine-resistant variant; adefovir-resistant mutant; tenofovir-resistant mutant; entecavir-resistant mutant, etc.); etc.

As used herein, "HBV genome" includes not only full-length genome (1 unit of genome), but also overlength HBV genome (>1 unit of genome, in other words, more than 1 unit of genome in length). The HBV genome contains all the information needed to build and maintain HBV replication. These genome sequences are available from any genotype in papers and GeneBank. "overlength HBV genome" or "over-full-length HBV genome" refers to a sequence comprising the full-length genome and a part of the genome, the sequence of which may vary according to the desired genomic unit and the specific HBV strain. Furthermore, methods of obtaining an over-full-length HBV genome and determining the genome sequence are described in the prior art, for example, in European Patent EP1543168.

In certain exemplary embodiments, the HBV refers to human HBV, and its genome contains four major overlapping open reading frames (ORFs), namely S-ORF, C-ORF, P-ORF, X-ORF. S-ORF is divided into S gene, pre-S2 region and pre-S1 region, each with its own initiator codon ATG, C-ORF is divided into C gene and pre-C region, each with its own initiator codon ATG, P-OFR is the longest reading frame, its starting segment overlaps with C-ORF, its middle segment overlaps with S-ORF, and its ending segment overlaps with X-ORF.

As used herein, "HBV genome fragment" refers to a portion of the HBV genome. The fragment may have at least 100, 200, 300, 400, 500, 600, 700, 800, 900, 1000, 1100, 1200, 1300, 1400, 1500, 1600, 1700, 1800, 1900, 2000, 2100, 2200, 2300, 2400, 2500, 2600, 2700, 2800, 2900, 3000, 3100 or 3200 consecutive nucleotides of the HBV genome. The fragment may also be a partial genome containing one or more genes contained in the HBV genome, for example, the fragment may be a nucleic acid encoding envelope protein, core/prenucleoprotein, x protein and/or HBV polymerase protein. Furthermore, the fragment may be a nucleic acid encoding one or more portions of the envelope protein, core/prenucleoprotein, x protein and/or polymerase protein of HBV.

As used herein, the terms "covalently closed circular DNA" and "cccDNA" are well known in the art and are used interchangeably herein. In general, "covalently closed circular DNA" or "cccDNA" refers to a replication intermediate of hepadnavirus genome and is a template for the synthesis of mRNA and pregenomic RNA of hepadnavirus.

As used herein, the term "cccDNA inhibitor" means it is capable of inhibiting the stability of cccDNA (i.e., reducing cccDNA stability), inhibiting the transcriptional activity of cccDNA (i.e., reducing the transcription of hepadnavirus mRNA that uses cccDNA as transcription template), and/or inhibiting cccDNA formation (i.e., no or less cccDNA formation).

As used herein, the term "variant" is used to refer to a polypeptide or polynucleotide having a certain degree of amino acid/nucleotide sequence identity to a parent polypeptide sequence or polynucleotide. The variant is similar to the parent sequence, but has at least one or several or more substitutions, deletions or insertions in its amino acid sequence or nucleotide sequence, such that it differs from the sequence of the parent polypeptide or polynucleotide. In some cases, the variant has been manipulated and/or engineered to contain at least one substitution, deletion or insertion in its amino acid sequence or nucleotide sequence, which makes it different from the parent sequence. In addition, the variant may retain the functional characteristics or activity of the parent polypeptide or parent polynucleotide, for example, retain at least 50%, 60%, 70%, 80%, 90%, 95%, 98% or 99% biological activity of the parent polypeptide or parent polynucleotide.

As used herein, the term "recombinant" DNA molecule refers to a DNA molecule formed by a laboratory method of genetic recombination (e.g., molecular cloning) to bring together genetic materials from multiple sources, and to generate a sequence that would not be found in biological organisms. The term "site-specific recombination" refers to a recombination between two nucleotide sequences, each of which contains at least one recognition site. "Site-specific" refers to a specific nucleotide sequence, which can be located at a specific location in the genome of a host cell. The nucleotide sequence may be endogenous to the host cell, and at the natural location in the host genome or at some other location in the genome, or it may be a heterologous nucleotide sequence previously inserted into the host cell genome by any of a variety of known methods.

As used herein, the term "recombinase" is a genetic recombinase, which is generally derived from bacteria and fungi, and catalyzes an orientation-sensitive DNA exchange reaction between short (30-40 nucleotides) target site sequences specific for each recombinase.

As used herein, the term "vector" refers to a nucleic acid delivery vehicle into which a polynucleotide can be inserted. When the vector can express the protein encoded by the inserted polynucleotide, the vector is called an expression vector. The vector can be introduced into a host cell by transformation, transduction or transfection, so that the genetic material elements carried by it can be expressed in the host cell. Vectors are well known to those skilled in the art and include, but are not limited to: plasmid; phagemid; cosmid; artificial chromosome, such as yeast artificial chromosome (YAC), bacterial artificial chromosome (BAC) or P1 derived artificial chromosome (PAC); phage such as λ phage or M13 phage, and animal virus. Animal viruses that can be used as vectors include, but are not limited to, retrovirus (including lentivirus), adenovirus, adeno-associated virus, herpesvirus (e.g., herpes simplex virus), poxvirus, baculovirus, papillomavirus, papovavirus (e.g., SV40). A vector may contain a variety of elements that control expression, including, but not limited to, promoter sequence, transcription initiation sequence, enhancer sequence, selection element, and reporter gene. Additionally, the vector may also contain an origin of replication site. Methods for introducing a vector and/or nucleic acid molecule carried thereby into a cell are known in the art, such as viral infection/transduction, conjugation, nanoparticle delivery, electroporation, particle gun technology, calcium phosphate precipitation, direct injection, etc. The choice of method generally depends on the type of cell being transfected and the environment in which the transfection takes place (i.e., in vitro, ex vivo, or in vivo). A general discussion of these methods can be found in Ausubel et al., Short Protocols in Molecular Biology, 3rd edition, Wiley & Sons, 1995.

As used herein, the term "host cell" refers to a cell into which a vector can be introduced, including, but not limited to, prokaryotic cell such as *E. coli* or *Bacillus subtilis,* fungal cell such as yeast cell or *Aspergillus,* insect cell such as S2 *Drosophila* cells or Sf9, or animal cell such as fibroblast, CHO cell, COS cell, NSO cell, HeLa cell, BHK cell, HEK 293 cell or human cell.

As used herein, the term "identity" refers to the match degree between two polypeptides or between two nucleic acids. When two sequences for comparison have the same monomer sub-unit of base or amino acid at a certain site (e.g., each of two DNA molecules has an adenine at a certain site, or each of two polypeptides has a lysine at a certain site), the two molecules are identical at the site. The percent identity between two sequences is a function of the number of identical sites shared by the two sequences over the total number of sites for comparison x 100. For example, if 6 of 10 sites of two sequences are matched, these two sequences have an identity of 60%. For example, DNA sequences: CTGACT and CAGGTT share an identity of 50% (3 of 6 sites are matched). Generally, the comparison of two sequences is conducted in a manner to produce maximum identity. Such alignment can be conducted by using a computer program such as Align program (DNAstar, Inc.) which is based on the method of Needleman, et al. (J. Mol. Biol. 48:443-453, 1970). The percent identity between two amino acid sequences can also be determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percentage of identity between two amino acid sequences can be determined by the algorithm of Needleman and Wunsch (J. Mol. Biol. 48:444-453 (1970)) which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6.

The twenty conventional amino acids referred to herein have been written following conventional usage. See, for example, Immunology-A Synthesis (2nd Edition, E. S. Golub and D. R. Gren, Eds., Sinauer Associates, Sunderland, Mass. (1991)), which is incorporated herein by reference. In the present invention, the terms "polypeptide" and "protein" have the same meaning and are used interchangeably. And in the present invention, amino acids are generally represented by one-letter and three-letter abbreviations well known in the art. For example, alanine can be represented by A or Ala.

### Beneficial effects of the present invention

The HBV cccDNA inhibitor screening model of the present invention uses split luciferase as a surrogate marker for HBV cccDNA detection. The detection is simple and short time-consuming, thus high-throughput drug screening can be realized. The detection can be used in many fields such as research, treatment, diagnosis, etc., and has broad application prospects and clinical value.

The embodiments of the present invention will be described in detail below with reference to the drawings and examples, but those skilled in the art will understand that the following drawings and examples are only used to illustrate the present invention, rather than limit the scope of the present invention. Various objects and advantageous aspects of the present invention will become apparent to those skilled in the art from the accompanying drawings and the following detailed description of the preferred embodiments.

### Brief Description of the Drawings

FIG. 1 shows the schematic diagram of PiggyBac transposon vector used for the construction of stable integration cell lines in Example 1.
FIG. 2 shows a schematic diagram of the HBV modification of the reporter model in which cccDNA generated during HBV replication process is indicated by HiBiT in Example 1.
FIG. 3 shows the viral replication and expression of HBV variant integrated with HiBiT tags of different sequences as assessed in Example 1.
FIG. 4 shows the evaluation of RG-Hibit16 cells for screening inhibitors that inhibit HBV cccDNA formation in Example 1.
FIG. 5 shows the schematic diagram and functional verification of HiBiT as the rcccDNA reporter model in Example 2.
FIG. 6 shows the screening of compounds targeting HBV cccDNA using RG-Hibit16 and G2-Rcccl in Example 3.
FIG. 7 shows verification of compounds with inhibitory effect on HBV cccDNA in the HepG2-hNTCP-2B1 infection model in Example 3.
FIG. 8 shows verification of compounds with promoting effect on HBV cccDNA in the HepG2-hNTCP-2B1 infection model in Example 3.

### Sequence information

The information on the partial sequences involved in the present invention is provided as follows.

| SEQ ID NO | Description |
|---|---|
| 1 | HBV 1.1-fold genome sequence |
| 2 | Amino acid sequence of HiBiT |
| 3 | Nucleotide sequence of HiBiT |
| 4 | Hibit16 insertion sequence |
| 5 | TRE3G promoter |
| 6 | Amino acid sequence of P2A |
| 7 | Nucleotide sequence of P2A |
| 8 | HBV genome variant sequence containing Hibit16 |
| 9 | Amino acid sequence of Tet-On 3G |
| 10 | Nucleotide sequence of Tet-On 3G |
| 11 | Amino acid sequence of iRFP |
| 12 | Nucleotide sequence of iRFP |
| 13 | Blasticidin-resistant gene |
| 14 | Rccc1a |
| | Sequence of 3' end region of C-ORF of HBV genome |
| 15 | Rccc1a |
| | Sequence of 5' end region of C-ORF of HBV genome |
| 16 | Rccc1a |
| | Sequence of HBV genome fragment in which C-ORF is removed |
| 17 | Rccc1a linear replicon sequence |
| 18 | Rccc1a recombinant cccDNA sequence |
| 19 | loxP sequence |
| 20 | Amino acid sequence of Cre recombinase |
| 21 | Nucleotide sequence of Cre recombinase |
| 22-30 | Primers, probes |

### EXAMPLES

The present invention will now be described with reference to the following examples, which are intended to illustrate, but not limit, the present invention.

Unless otherwise specified, the molecular biology experimental methods and immunoassays used in the present invention are performed by basically referring to the methods described in J. Sambrook et al., Molecular Cloning: A Laboratory Manual, 2nd Edition, Cold Spring Harbor Laboratory Press, 1989, and F. M. Ausubel et al., Refined Molecular Biology Laboratory Manual, 3rd Edition, John Wiley & Sons, Inc., 1995; and the restriction enzymes were used according to the conditions recommended by the product manufacturer. Those skilled in the art appreciate that the examples describe the present invention by way of example and are not intended to limit the scope of the invention as claimed.

The main detection methods involved in the following examples are described as follows:
Detection of HiBiT: Nano Glo HiBiT Extracellular Detection System (Cat. No. N2421) of Promega Corporation was used for HiBiT detection, and the detection steps were carried out according to the kit instructions.
Detection of HBsAg/HBeAg: The detection procedures of hepatitis B surface antigen (chemiluminescence method CLEIA, product standard number: YZB/Guo 0346-2014) and e antigen (enzyme-linked immunosorbent assay ELISA, product standard number: YZB/Guo 0216-2013) were both carried out according to the detection methods of the kits of Beijing Wantai Company.
Detection of HBV DNA: For the HBV DNA extraction, after the collected cells were washed with PBS, the virus DNA & RNA extraction kit (Beijing GenMagBio) was used for automatic extraction at the nucleic acid extraction workstation. For the HBV DNA quantification, Premix Ex TaqTM (Takara) was used for the probe method, with the instrument of Roche's LightCycler^{®} 96, and the primer sequences used are shown in Table 1.

**Table 1: PCR primer sequences**

| Primer | Sequence (5'-3') | SEQ ID NO: |
|---|---|---|
| HBV-F | TTTCACCTCTGCCTAATCAT | 22 |
| HBV-R | TCAGAAGGCAAAAAAGAGAGTAACTC | 23 |
| HBV-Probe | HEX-CCTTGGGTGGCTTTGGGGCATGGA-BHQ1 | 24 |
| cccDNA-Probe | FAM-ACCGTGAACGCCCACCGAATGTTGC-BHQ1 | 25 |
| cccDNA-F | TGCACTTCGCTTCACCT | 26 |
| cccDNA-R | AGGGGCATTTGGTGGTC | 27 |
| mt4987F | CCCAGCTACGCAAAAT | 28 |
| mt5106R | AATGCGGTAGTAGTTAGGATA | 29 |
| mt5010-Probe | HEX-CATACTCCTCAATTACCCACATAG-BHQ1 | 30 |

Detection of HBV cccDNA: The modified Hirt method was used for HBV cccDNA extraction, with Tiangen Plasmid Mini Kit. The lysis buffers involved were Buffer I (50mM Tris, 10mM EDTA, pH 7.5), Buffer II (1.2 % SDS), Buffer III (3M CsCl, 1M potassium acetate, 0.67M acetic acid), which were used to replace P1, P2 and P3 in the Tiangen Plasmid Mini Kit respectively. The extraction steps and methods were performed by referring to the plasmid extraction method of the kit. The primers used for fluorescence quantification are shown in Table 1. The instrument used is Roche's LightCycler^{®} 96. HBV cccDNA and mitochondrial DNA (mtDNA) were quantified respectively, and the relative values of HBV cccDNA and mtDNA were calculated.

The steps for the detection of HBV DNA by DNA immunoblotting were as follows. DNA extraction: cells were washed once with PBS after treatment; NET Buffer (50mM Tris-pH8.0, 1mM EDTA, 100mM NaCl, 0.5% NP-40) was added to lyse the cells at 4°C for 1 h; the cell lysate supernatant was collected, added with 33 µg/mL Micrococcal nuclease and 6mM CaCl₂ at final concentration, and allowed to stay in 37°C water bath for 30 minutes; added with 25 mM EDTA at final concentration, and allowed to stay in 65°C water bath for 15 minutes; added with 200 µg/mL proteinase K and 0.5% SDS at final concentration, and allowed to stay in 50°C water bath for 12 h; and DNA was extracted with phenol chloroform. Detection: DNA was separated by electrophoresis on 1.2% agarose for 2 h, then the gel was treated with 0.2N HCl, 0.5MNaOH/1.5M NaCl and 1M Tris-HCl in turn to denature the DNA, and then the nucleic acid was transferred to nylon membrane by a vacuum blotter. The nucleic acid was fixed by UV cross-linking, then subjected to pre-hybridization and hybridization, excess probes were washed off, blocking solution was added for blocking, then Anti-Dig-Ap antibody was added, CDP-star was finally added to develop color, and the target strip was detected by continuous exposure.

### Example 1: Construction of reporter model using HiBiT to indicate cccDNA produced during HBV replication

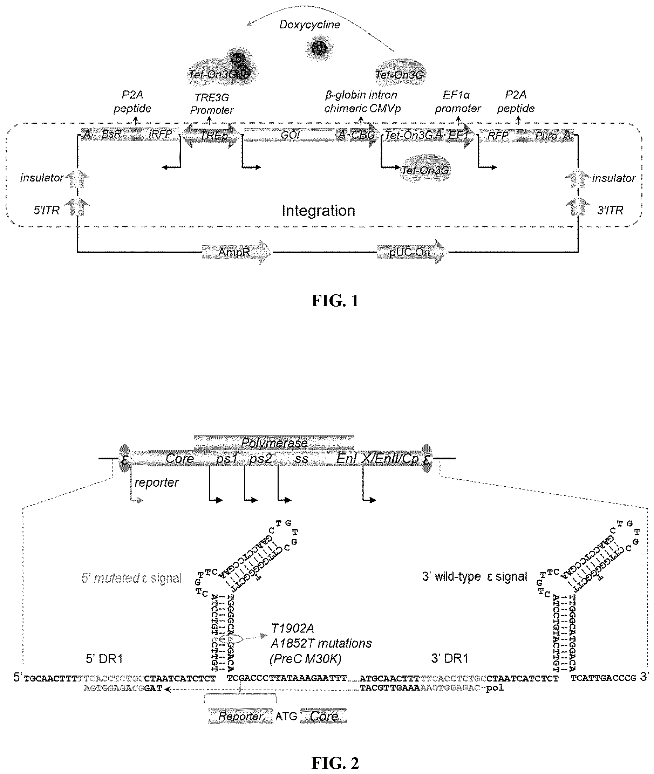

The PB-CMV-MCS-EF1α-RedPuro (Cat.#PB514B-1) of the PiggyBac transposon system was used as a vector in this example; when the vector was co-transfected with PiggyBac transposase (System Biosciences, PB210PA-1), the sequence between the two "ITR sequences" on the vector plasmid could be integrated into the genome of the cell to achieve the integration of the target gene. In order to achieve the regulatory expression of HBV, we replaced the "CMV Promoter" on the vector with "TRE3G Promoter" (Takara, Tet-On 3G Inducible Expression System), the promoter required Doxycycline combined with Tet-On 3G Transactivator to start transcription, so the expression of the target protein could be regulated with Doxycycline. In order to avoid the loss of part of the target sequence during integration, we selected the TRE3G promoter with bidirectional promotion activity, introduced iRFP fluorescent marker and Blasticidin resistance selection marker at the N-terminus, and used dual resistance and dual fluorescence as the screening conditions of integrated cells. Since the Tet-On 3G protein was necessary for the transcription of TRE3G promoter, we introduced an expression cassette into the vector to express the Tet-On 3G protein. The final vector is shown in FIG. 1, in which the sequence in the red dashed box is the sequence integrated into the cell genome. "GOI" represents the ligated target gene (Gene of interest), and the ligated sequence in this example is the HBV 1.1-fold genome sequence into which the reporter gene is inserted.

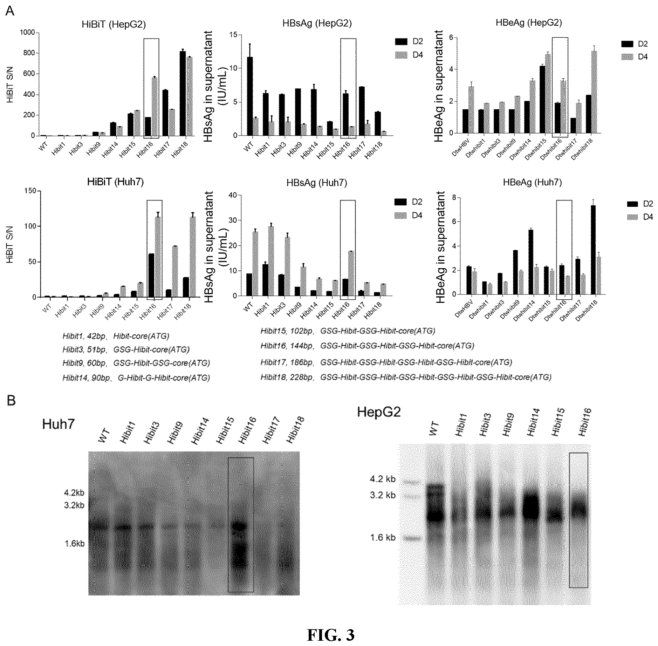

The open reading frames of the HBV genome are highly overlapping, so the modification of HBV has strict restrictions on the insertion position of foreign genes. In this example, HiBiT sequences with different copy numbers and connected by different linker peptides were inserted between the pre core and the core to prepare HBV variants containing HiBiT. The schematic diagram of the insertion is shown in FIG. 2. The HiBiT signal, viral protein expression and viral replication of these insertion mutations were verified. Different HBV variants were transfected in hepatoma cell lines HepG2 and Huh7, respectively, the HiBiT signal, the expression of HBV antigens HBsAg and HBeAg (A) in the cell supernatant were detected, and the viral replication (B) was detected by Southern Blot, and the results were shown in FIG. 3. It could be seen that Hibit16, i.e., insertion mutation (SEQ ID NO: 4) with insertion of 3 copies of HiBiT tag, was a better choice; a decrease in HiBiT copy number might result in weak HiBiT signal, while an increase in the HiBiT copy number might affect the expression or replication of viral proteins. For the comparison of different linker peptides, insertion of the peptide "GSG" before and after the HiBiT sequence was better than insertion of the peptide "G" at one end or introduction of no linker peptide. Therefore, in this example, HepaRG cells stably integrated with HBV variant Hibit16 were constructed to screen for inhibitors that could inhibit the formation of cccDNA.

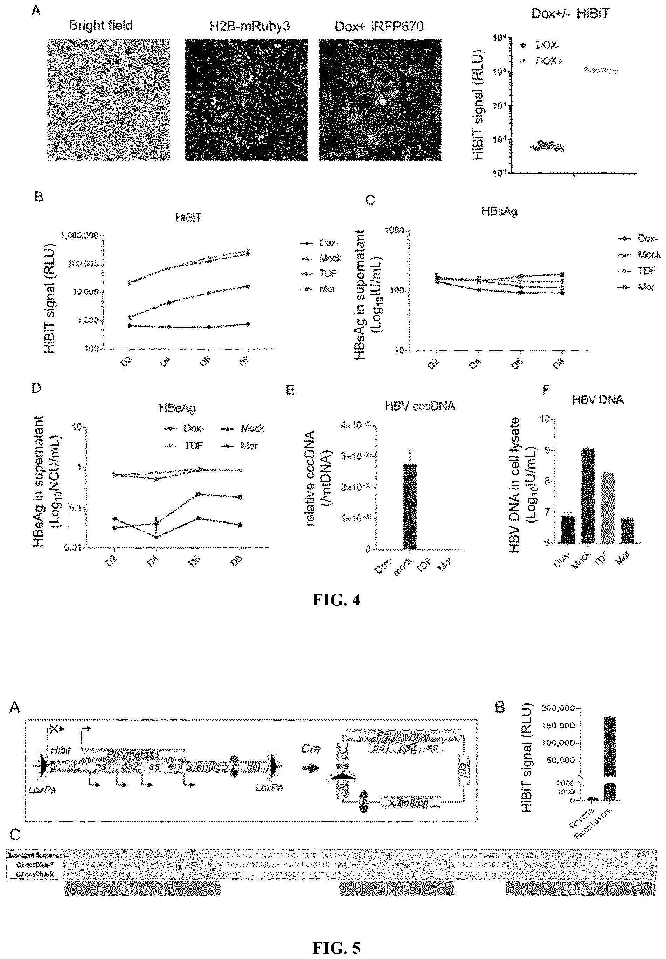

HepaRG-Hibit16 cells were obtained by transfecting HepaRG cells with Hibit16 plasmid and PiggyBac transposase, integrating the HBV variant sequence (Hibit16) and selection markers into the genome of the cells, and screened by puromycin resistance and red fluorescent marker; Doxycycline could activate TRE3G promoter to initiate the expression of iRFP670, as well as the transcription and replication of HBV, thereby generating HiBiT, as shown in FIG. 4A. To evaluate the function of HBV inhibitors in the cells, the cells were first plated, and Doxycycline was added to induce viral transcription and replication while different HBV inhibitors were added for intervention. The cell supernatant was collected every 2 days, the medium was replaced, and the HiBiT signal, HBsAg, HBeAg in the cell supernatant, and the HBV DNA and HBV cccDNA in the cell lysate were detected, and the responses of the cells to HBV inhibitors were shown in FIGS. 4B-F. Tenofovir (tenofovir disoproxil fumarate, TDF) is a nucleotide reverse transcriptase inhibitor, and Morphothiadin (abbreviation: Mor) is an assembly regulator of core particles, which are already in the clinical Phase II/III evaluation stage, and both can inhibit the formation of cccDNA. In the cell model constructed in this study, TDF and Mor could inhibit the formation of cccDNA, and the expression level of HiBiT in the Mor-treatment group was significantly lower than that in the control group, indicating that this model could be used to screen for inhibitors such as Mor that inhibited the formation of cccDNA.

### Example 2: Construction of reporter model for recombinant rcccDNA indicated by HiBiT

In this example, a reporter model using HiBiT to indicate recombinant cccDNA was constructed. Recombinant cccDNA, i.e., rcccDNA, was a closed circular DNA formed by circularizing linear HBV DNA by Cre/loxP recombinase system. We constructed the HBV variant shown in FIG. 5A, in which the C-terminal partial sequence of the HBV core ORF (SEQ ID NO: 14) was placed at the N-terminal of the entire replicon, while the N-terminal partial sequence of the core ORF and the promoter of core (SEQ ID NO: 15) was placed at the C-terminus of the replicon, the sequence of the reporter gene HiBiT was placed at the N-terminus of the replicon, and the loxP sequence (SEQ ID NO: 19) was added before and after the replicon. Without the expression of Cre recombinase, HiBiT lacked a promoter and could not be expressed, but after the formation of rcccDNA, HiBiT could use the promoter of core to express HBeAg and HBcAg fused with the HiBiT tag. Therefore HiBiT could be used as a surrogate marker for rcccDNA detection. The HBV variant used the PiggyBac transposon system as vector, which facilitated the integration of the target gene, that was, the HBV variant with the integrated reporter gene, into the cell genome to construct an integrated cell line, and the clone was named as Rccc1a. The plasmid was transfected into HepG2 cells, after 6 h, the medium was changed for infection with adenovirus Adv-Cre to express Cre recombinase (SEQ ID NO: 20), and HiBiT in the cell supernatant was detected after 48 h; the cells were lysed, the partial sequences of rcccDNA before and after the loxP sequence were amplified by using the lysate as template, to verify whether the expected rcccDNA was formed, and the amplification primers and sequencing primers both were cccDNA-F and cccDNA-R in Table 1. FIG. 5B showed the HiBiT detection results in the cell supernatants of the HepG2 cells transfected with Rccc1a with or without Cre recombinase expression, and FIG. 5C showed the sequencing results of rcccDNA formed after expression of Cre recombinase of HepG2 cells transfected with Rccc1a. The above results showed that Cre recombinase could make the expected rcccDNA to be generated, and initiate the expression of the protein with HiBiT tag.

### Example 3: Application of reporter model for screening cccDNA inhibitors

The reporter model prepared in Example 1 comprised an HBV variant integrated with 3 repeats of HiBiT sequence. The mRNA transcribed directly from the HBV variant as a template lacked the initiation codon for HiBiT expression, and could not translate the protein attached to the HiBiT tag. Only after the pgRNA transcribed from the HBV variant was reversely transcribed to form cccDNA, the mRNA transcribed from cccDNA as a template could translate the protein attached to the HiBiT tag, so the expression level of HiBiT could be used to indicate the formation of HBV cccDNA. In the reporter model prepared in Example 2, the HiBiT sequence was inserted in the middle of the HBV core sequence, the N-terminus sequence and C-terminus sequence of the core were respectively attached to the C-terminus and N-terminus of the HBV replicon, and loxP sequences were ligated to both ends of the HBV replicon, so that in the absence of Cre recombinase expression, the HiBiT tag lacked a promoter and could not be expressed; but with the expression of Cre recombinase, it could mediate the recombination of double-stranded DNA, so that the linear HBV genome DNA formed a closed circular DNA, and after the circular DNA was formed, the HiBiT tag could use the endogenous promoter of HBV to initiate the expression of the protein attached to the HiBiT tag, so the signal of the HiBiT tag could be used to indicate the formation of recombinant HBV cccDNA, i.e., HBV rcccDNA.

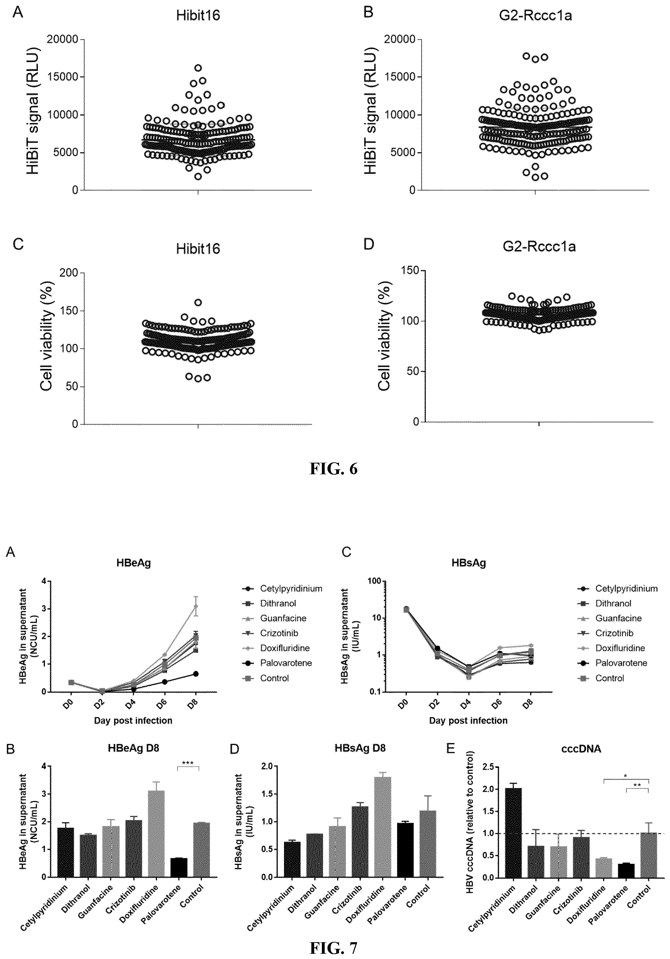

In this example, the reporter model of Example 1 (HepaRG-Hibit16 integrated with Hibit16 constructed based on HepaRG cells) and the reporter model of Example 2 (HepG2-Rccc1a integrated with Rccc1a constructed based on HepG2) were investigated for screening cccDNA inhibitors. These two cells were used to evaluate the potential of 189 drugs to inhibit cccDNA, including clinical drugs for different diseases or drugs in the clinical research stage. The screening work was carried out in a 96-well cell culture plate, RG-Hibit16 cells were plated at a cell density of 15,000/well, and G2-Rccc1a cells were plated at a cell density of 35,000/well. RG-Hibit16 cells were treated with drugs 1 week after plating. For RG-Hibit16, different compounds were added for treatment when Dox was added to induce virus expression; the next day after G2-Rccc1a cells were plated, they were firstly infected with adenovirus Adv-Cre to express Cre recombinase protein, and different compounds were added 2 days later for treatment, and all compounds were diluted to 1 µM at the final concentration, 200 µL per well; new medium was replaced every 2 days, the expression of HiBiT in the cell supernatant was detected after 4 days of compound treatment, and CCK8 detection reagent was added at a ratio of 10:1 to detect the cytotoxicity of the compounds. The results of using RG-Hibit16 cells and G2-Rccc1a cells in the compound screening were shown in FIG. 6. Among the 189 compounds, the 3 compounds that most significantly inhibited the expression level of HiBiT in RG-Hibit16 cells were Cetylpyridinium, Guanfacine and Palovarotene; the 4 compounds that most significantly inhibited the expression level of HiBiT in G2-Rccc1a cells were Crizotinib, Doxifluridine, Palovarotene and Dithranol; and these 6 drugs had no obvious cytotoxicity to the two kinds of cells according to the detection results of CCK8. Palovarotene showed the inhibitory effect on HBV cccDNA in both models.

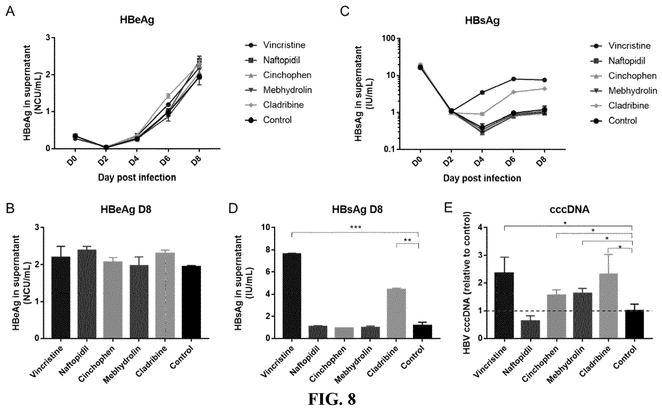

We further verified the functions of these 6 compounds in the HepG2-hNTCP-2B 1 infection model. HepG2-hNTCP-2B 1 was the single clone 2B 1 selected for the highest susceptibility to HBV which is made by overexpressing hNTCP in HepG2 cells. In the evaluation by this model, the cells were firstly infected with HBV virus, washed with PBS to remove the residual virus on the next day, different compounds were added after 2 days, and then the cell supernatant was collected and fresh medium was replaced every 2 days, the viral antigen in the cell supernatant was detected, the cells were lysed after 8 days of infection, the intracellular HBV cccDNA was detected, and the evaluation results were shown in FIG. 7. The inhibitory effect of Palovarotene on HBsAg was relatively poor, but the inhibition rates against HBsAg and HBV cccDNA were all more than 60%, which were better than the other five compounds. In addition, Doxifluridine also had a significant inhibitory effect on cccDNA in this infection model. According to the evaluation results of RG-Hibit16 cells and G2-Rccc1a cells, we also selected 5 compounds that had up-regulated effects on HBV cccDNA in both models, namely: Vincristine, Naftopidil, Cinchophen, Mebhydrolin and Cladribine. The functions of these five compounds were verified in the HepG2-hNTCP-2B 1 infection model, and the evaluation strategy was the same as that of the aforementioned inhibitor. The results are shown in FIG. 8. The five compounds had weak promotion effect on HBeAg; Vincristine and Cladribine could significantly up-regulate the expression level of HBsAg; and Vincristine, Cinchophen, Mebhydrolin and Cladribine could up-regulate the intracellular cccDNA level. The verification results of the infection model showed that the two cccDNA reporter models constructed in this study could be used to screen compounds that could promote or inhibit cccDNA. Although some of the compounds screened did not show corresponding effects in the infection model, the range of candidate compounds was greatly reduced, so that they could be used for preliminary screening of compounds targeting HBV cccDNA.

Although specific embodiments of the present invention have been described in detail, those skilled in the art will appreciate that various modifications and changes can be made to the details in light of all the teachings that have been published, and that these changes are all within the scope of the present invention. The full division of the invention is given by the appended claims and any equivalents thereof.

## Claims

1. An isolated nucleic acid molecule, which comprises a variant of HBV genome sequence, wherein the variant comprises: a HBV genome fragment comprising C-ORF, S-ORF and P-ORF, the C-ORF comprises an exogenous insertion sequence between precore and core genes, and the exogenous insertion sequence comprises a nucleotide sequence encoding a first fragment of luciferase; the first fragment of luciferase is capable of binding to a corresponding second fragment of luciferase of a luciferase fragment complementation assay (LFCA), and thereby generating luciferase activity.

2. The isolated nucleic acid molecule according to claim 1, wherein the HBV genome fragment further comprises an X-ORF.

3. The isolated nucleic acid molecule according to claim 1 or 2, wherein the variant comprises the exogenous insertion sequence between the precore and core genes of the HBV genome sequence.

4. The isolated nucleic acid molecule according to any one of claims 1 to 3, wherein the first fragment of luciferase is a complementary small fragment capable of binding to LgBiT, such as HiBiT or SmBiT; the second fragment of luciferase is LgBiT;
preferably, the first fragment of luciferase is HiBiT and the second fragment of luciferase is LgBiT.

5. The isolated nucleic acid molecule according to claim 4, wherein the exogenous insertion sequence comprises multiple copies of the nucleotide sequence encoding the first fragment of luciferase (e.g., HiBiT) in tandem repeats;
preferably, the exogenous insertion sequence comprises three copies of the nucleotide sequence encoding the first fragment of luciferase (e.g., HiBiT) in tandem repeats.

6. The isolated nucleic acid molecule according to claim 5, wherein each copy of the multiple copies of the nucleotide sequence encoding the first fragment of luciferase (e.g., HiBiT) in tandem repeats comprises at its 5'-end a sequence encoding a linker peptide (e.g., a flexible peptide linker).

7. The isolated nucleic acid molecule according to claim 1, wherein the exogenous insertion sequence comprises the sequence set forth in SEQ ID NO:4.

8. The isolated nucleic acid molecule according to any one of claims 1 to 7, wherein the HBV genome is a full-length genome, or an overlength genome, such as a 1.1-fold genome or a 1.3-fold genome;
preferably, the HBV genome comprises the sequence set forth in SEQ ID NO: 1.

9. The isolated nucleic acid molecule according to any one of claims 1 to 8, which further comprises an inducible promoter operably linked to the exogenous insertion sequence;
preferably, the inducible promoter is a TRE3G promoter, or comprises one or more repeats of Tet operator sequence (TetO);
preferably, the inducible promoter has bidirectional activation activity, for example is a TRE3G promoter with bidirectional activation activity.

10. The isolated nucleic acid molecule according to claim 9, wherein the isolated nucleic acid molecule further comprises a reporter gene operably linked to the inducible promoter;
preferably, the reporter gene is in the opposite direction to the exogenous insertion sequence;
preferably, the reporter gene is selected from fluorescent protein genes (e.g., iRFP) and/or antibiotic resistance genes (e.g., Blasticidin);
preferably, the reporter gene comprises a fluorescent protein gene and an antibiotic resistance gene;
preferably, the fluorescent protein gene and the antibiotic resistance gene are optionally linked by a nucleotide sequence encoding a self-cleaving peptide (e.g., P2A, E2A, F2A or T2A).

11. The isolated nucleic acid molecule according to claim 9 or 10, wherein the isolated nucleic acid molecule comprises the sequence set forth in SEQ ID NO:8.

12. A recombinant HBV cccDNA, which comprises the isolated nucleic acid molecule according to any one of claims 1 to 11;
preferably, the recombinant HBV cccDNA comprises the variant of HBV genome sequence as defined in any one of claims 1 to 11;
preferably, the recombinant HBV cccDNA is formed by circularization of the isolated nucleic acid molecule according to any one of claims 1 to 11.

13. An expression system, which comprises the isolated nucleic acid molecule according to any one of claims 9 to 11 as a first nucleic acid sequence, and comprises a second nucleic acid sequence, wherein the second nucleic acid sequence comprises a nucleotide sequence coding a transactivator corresponding to the inducible promoter contained in the first nucleic acid sequence;
preferably, the transactivator is selected from Tet-On 3G transactivator, rTetR, rtTA;
preferably, the second nucleic acid sequence further comprises an expression control element, such as a promoter (e.g., a constitutive promoter) and/or an enhancer, that is operably linked to the nucleotide sequence encoding the transactivator.

14. The expression system according to claim 13, wherein the first nucleic acid sequence comprises a TRE3G promoter as an inducible promoter, and the second nucleic acid sequence comprises a nucleotide sequence encoding a Tet-On 3G transactivator;
preferably, the TRE3G promoter comprises the sequence set forth in SEQ ID NO:5;
preferably, the nucleotide sequence encoding the Tet-On 3G transactivator comprises the sequence set forth in SEQ ID NO:10.

15. A vector, which comprises the isolated nucleic acid molecule according to any one of claims 1 to 11, or the expression system according to claim 13 or 14.

16. The vector according to claim 15, wherein the vector comprises the expression system according to claim 13 or 14, wherein the first nucleic acid sequence and the second nucleic acid sequence are provided on the same or different vectors;
preferably, the first nucleic acid sequence and the second nucleic acid sequence are provided on the same vector.

17. The vector according to claim 15 or 16, wherein the vector is a transposon vector, such as a PiggyBac transposon vector;
preferably, the first nucleic acid sequence and the second nucleic acid sequence are located between the two ITR sequences of the transposon vector.

18. A co-transfection system, which comprises the vector according to any one of claims 15 to 17, and a transposase expression vector;
preferably, the transposase expression vector is a PiggyBac transposase expression vector.

19. A host cell, which comprises the isolated nucleic acid molecule according to any one of claims 1 to 11, or the recombinant cccDNA according to claim 12, or the expression system according to claim 13 or 14, or the vector according to any one of claims 15 to 17, or the co-transfection system according to claim 18;
preferably, the host cell is selected from eukaryotic cells derived from hepatocyte, such as hepatoma cell or hepatocyte; preferably, the host cell is selected from HepaRG, HepG2 or Huh7.

20. The host cell according to claim 19, wherein the host cell comprises the expression system according to claim 13 or 14 in its genome;
preferably, the host cell is capable of stably expressing an HBV cccDNA formed from the variant of HBV genome sequence in the presence of an inducer (e.g., Doxycycline) corresponding to the inducible promoter and transactivator.

21. A kit, which comprises the isolated nucleic acid molecule according to any one of claims 1 to 11, or the expression system according to any one of claims 13 or 14, or the vector according to any one of claims 15 to 17, or the co-transfection system according to claim 18, or the host cell according to claim 19 or 20;
preferably, the kit comprises: the vector according to any one of claims 15 to 17, or the co-transfection system according to claim 18;
preferably, the kit comprises: the host cell according to claim 19 or 20;
preferably, the kit further comprises a LgBiT protein and optionally a luciferase substrate;
preferably, the kit further comprises an inducer (e.g., Doxycycline) corresponding to the inducible promoter and transactivator.

22. A method for screening a HBV cccDNA inhibitor, which comprises:
(1) providing the host cell according to claim 20;
(2) contacting an inducing agent with the host cell, wherein the inducing agent is an inducer (e.g., Doxycycline) corresponding to the inducible promoter and transactivator contained in the host cell;
(3) contacting a test agent with the host cell; wherein, steps (2) and (3) can be performed simultaneously or in any order;
(4) detecting a level of the first fragment of luciferase in a cell supernatant of the host cell.

23. The method according to claim 22, wherein, step (1) comprises the following steps:
(1a) introducing the first nucleotide sequence and the second nucleotide sequence in the expression system according to claim 13 or 14 into the host cell, wherein the first nucleotide sequence and the second nucleotide sequence are provided on the same or different expression vectors, and the first nucleic acid sequence is the isolated nucleic acid molecule according to any one of claims 9 to 11;
(1b) culturing the host cell;
preferably, the host cell is selected from eukaryotic cells derived from hepatocytes, such as hepatoma cell or hepatocyte; preferably, the host cell is selected from HepaRG, HepG2 or Huh7;
preferably, in step (1a), the expression vector is a transposon vector (e.g., PiggyBac transposon vector), and the step further comprises: introducing a transposase expression vector (e.g., PiggyBac transposase expression vector) into the host cell;
preferably, the step (1) further comprises: (1c) identifying and selecting a host cell with the expression system according to claim 13 or 14 integrated in its genome; preferably, whether the expression system has been integrated into the genome of the host cell is identified by detecting a reporter gene contained in the first nucleic acid sequence.

24. The method according to claim 22 or 23, wherein, in step (4), the level of the first fragment of luciferase is detected by a luciferase fragment complementation assay;
preferably, the detection is carried out with a second fragment of luciferase that is complementary to the first fragment of luciferase;
preferably, the first fragment of luciferase is a complementary small fragment capable of binding to LgBiT, such as HiBiT or SmBiT, and the second fragment of luciferase is a LgBiT protein; preferably, the first fragment of luciferase is HiBiT, and the second fragment of luciferase is the LgBiT protein.

25. The method according to any one of claims 22 to 24, which further comprises the steps of:
(5) comparing the detection result of step (4) with the level of the first fragment of luciferase detected in the absence of the test agent;
wherein, if the detection result in step (4) is lower than the detection result in the absence of the test agent, it indicates that the test agent is an HBV cccDNA inhibitor.

26. An isolated nucleic acid molecule, which comprises a variant of HBV genome sequence, the variant comprising from the 5' to 3':
(i) a nucleotide sequence encoding a first fragment of luciferase; the first fragment of luciferase is capable of binding to a corresponding second fragment of luciferase of a luciferase fragment complementation assay (LFCA), thereby producing a luciferase activity;
(ii) a sequence of the 3' end region of C-ORF of HBV genome;
(iii) an HBV genome fragment containing S-ORF and P-ORF;
(iv) a sequence of the 5' end region of C-ORF of HBV genome, which can form a complete C-ORF sequence with the sequence described in (ii);
and, the variant is located between two site-specific recombinase recognition sequences arranged in the same orientation.

27. The isolated nucleic acid molecule according to claim 26, wherein the HBV genome is a full-length genome, or an overlength genome, such as a 1.1-fold genome or a 1.3-fold genome;
preferably, the HBV genome comprises the sequence set forth in SEQ ID NO: 1.

28. The isolated nucleic acid molecule according to claim 26 or 27, wherein the sequence of (iii) further comprises an X-ORF;
preferably, the sequence of (iii) comprises a HBV genome fragment from which C-ORF is removed;
preferably, the sequence of (iii) comprises the sequence set forth in SEQ ID NO: 16.

29. The isolated nucleic acid molecule according to any one of claims 26 to 28, wherein the sequence of (ii) comprises a core gene, and the sequence of (iv) comprises a pre-core gene;
preferably, the sequence of (ii) comprises the sequence set forth in SEQ ID NO: 14;
preferably, the sequence of (iv) comprises the sequence set forth in SEQ ID NO: 15.

30. The isolated nucleic acid molecule according to any one of claims 26 to 29, wherein the site-specific recombinase recognition sequences are selected from a loxP sequence or a FRT sequence.

31. The isolated nucleic acid molecule according to claims 26 to 30, wherein the first fragment of luciferase is a complementary small fragment capable of binding to LgBiT, such as HiBiT or SmBiT, and the second fragment of luciferase is LgBiT;
preferably, the first fragment of luciferase is HiBiT and the second fragment of luciferase is LgBiT.

32. The isolated nucleic acid molecule according to any one of claims 26 to 31, which comprises the sequence set forth in SEQ ID NO: 17.

33. A recombinant HBV cccDNA, which is formed by circularization of the variant of HBV genome sequence in the isolated nucleic acid molecule according to any one of claims 26 to 32;
preferably, the recombinant HBV cccDNA is formed by circularization of the isolated nucleic acid molecule according to any one of claims 26 to 32 in the presence of a site-specific recombinase (e.g., Cre recombinase or FLP recombinase) corresponding to the site-specific recombinase recognition sequence;
preferably, the recombinant HBV cccDNA comprises C-ORF, S-ORF, P-ORF, and the C-ORF comprises a nucleotide sequence encoding the first fragment of luciferase (e.g., HiBiT);
preferably, the recombinant HBV cccDNA further comprises an X-ORF;
Preferably, the recombinant HBV cccDNA comprises: a C-ORF comprising a nucleotide sequence encoding the first fragment of luciferase (e.g., HiBiT), and a HBV genome fragment from which the C-ORF has been removed;
preferably, the recombinant cccDNA comprises the sequence set forth in SEQ ID NO: 18.

34. A vector, which comprises the isolated nucleic acid molecule according to any one of claims 26 to 32.

35. The vector according to claim 34, which is a transposon vector, such as a PiggyBac transposon vector;
preferably, the isolated nucleic acid molecule is located between the two ITR sequences of the transposon vector.

36. A co-transfection system, which comprises the vector according to claim 35, and a transposase expression vector;
preferably, the transposase expression vector is a PiggyBac transposase expression vector.

37. A host cell, which comprises the isolated nucleic acid molecule according to any one of claims 26 to 32, or the recombinant cccDNA according to claim 33, or the vector according to claim 34 or 35, or the co-transfection system according to claim 36;
preferably, the host cell is selected from eukaryotic cells derived from hepatocyte, such as hepatoma cell or hepatocyte; preferably, the host cell is selected from HepaRG, HepG2 or Huh7.

38. The host cell according to claim 37, wherein the host cell comprises the isolated nucleic acid molecule according to any one of claims 26 to 32 in its genome;
preferably, the host cell is capable of stably expressing the recombinant HBV cccDNA formed by circularization of the variant of HBV genome sequence in the presence of a site-specific recombinase (e.g., Cre recombinase or FLP recombinase) corresponding to the site-specific recombinase recognition sequence.

39. A kit, which comprises the isolated nucleic acid molecule according to any one of claims 26 to 32, or the recombinant cccDNA according to claim 33, or the vector according to claim 34 or 35, or the co-transfection system according to claim 36, or the host cell according to claim 37 or 38;
preferably, the kit comprises: the vector according to claim 34 or 35, or the co-transfection system according to claim 36;
preferably, the kit comprises: the host cell according to claim 37 or 38;
preferably, the kit further comprises a LgBiT protein and optionally a luciferase substrate;
preferably, the kit further comprises a recombinase (e.g., Cre recombinase or FLP recombinase) or a recombinase (e.g., Cre recombinase or FLP recombinase) expression vector.

40. A method for screening an HBV cccDNA inhibitor, comprising:
(1) providing the host cell according to claim 38;
(2) introducing a recombinase or a recombinase expression vector into the host cell, wherein the recombinase corresponds to the site-specific recombinase recognition sequence contained in the host cell;
(3) contacting a test agent with the host cell;
(4) detecting a level of the first fragment of luciferase in a cell supernatant of the host cell.

41. The method according to claim 40, wherein step (1) comprises the steps of:
(1a) introducing the isolated nucleic acid molecule according to any one of claims 26 to 32 or the vector according to claim 34 or 35 into the host cell;
(1b) culturing the host cell;
preferably, the host cell is selected from eukaryotic cells derived from hepatocyte, such as hepatoma cell or hepatocyte; preferably, the host cell is selected from HepaRG, HepG2 or Huh7;
preferably, in step (1a), the expression vector is a transposon vector (e.g., PiggyBac transposon vector), and the step further comprises: introducing a transposase expression vector (e.g., PiggyBac transposase expression vector) into the host cell.

42. The method according to claim 40 or 41, wherein, in step (4), the level of the first fragment of luciferase is detected by a luciferase fragment complementation assay;
preferably, the detection is performed with a second fragment of luciferase that is complementary to the first fragment of luciferase;
preferably, the first fragment of luciferase is a complementary small fragment capable of binding to LgBiT, such as HiBiT or SmBiT, and the second fragment of luciferase is a LgBiT protein; preferably, the first fragment of luciferase is HiBiT, the second fragment of luciferase is the LgBiT protein.

43. The method according to any one of claims 40 to 42, which further comprises the steps of:
(5) comparing the detection result of step (4) with a level of the first fragment of luciferase detected in the absence of the test agent;
wherein, if the detection result in step (4) is lower than the detection result in the absence of the test agent, it indicates that the test agent is an HBV cccDNA inhibitor.

44. Use of the isolated nucleic acid molecule according to any one of claims 1 to 11, or the expression system according to claim 13 or 14, or the vector according to any one of claims 15 to 17, or the co-transfection system according to claim 18, or the host cell according to claim 19 or 20, or the kit according to claim 21, or the isolated nucleic acid molecule according to any one of claims 26 to 32, or the recombinant cccDNA according to claim 33, or the vector according to claim 34 or 35, or the co-transfection system according to claim 36, or the host cell according to claim 37 or 38, or the kit according to claim 39, for screening an HBV cccDNA inhibitor.
